# EUROPÄISCHE PATENTSCHRIFT

(11) **EP 2 585 217 B1**
(45) Veröffentlichungstag und Bekanntmachung des Hinweises auf die Patenterteilung: **14.01.2015**
(21) Anmeldenummer: 11726069.5
(22) Anmeldetag: 08.06.2011
(51) Int. Cl.: B01L 3/00, C12M 1/26, C12M 1/12, G01N 1/02, C12Q 1/04

(54) **VORRICHTUNG UND VERFAHREN ZUR ANALYSE EINER KONTAMINIERTEN OBERFLÄCHE**
APPARATUS AND METHOD FOR ANALYSING A CONTAMINATED SURFACE
DISPOSITIF ET PROCÉDÉ D'ANALYSE D'UNE SURFACE CONTAMINÉE

(30) Priorität: 24.06.2010 DE 102010024933
(43) Veröffentlichungstag der Anmeldung: 01.05.2013
(73) Patentinhaber: Sartorius Stedim Biotech GmbH, 37079 Göttingen (DE)
(72) Erfinder: PFLANZ, Karl, 37130 Gleichen (DE)
(86) Internationale Anmeldenummer: PCT/EP2011/002794
(87) Internationale Veröffentlichungsnummer: WO 2011/160773

(56) Entgegenhaltungen:
- DE-U1-202009 016 410
- JP-A- 2008 193 919

## Beschreibung

Die Erfindung betrifft eine Vorrichtung zur Analyse einer kontaminierten Oberfläche mit einer Transfereinrichtung mit einem porösen scheibenförmigen Medium und mit einer Analyseeinrichtung. Weiterhin betrifft die Erfindung ein Verfahren zur Analyse einer kontaminierten Oberfläche unter Verwendung der vorgenannten Vorrichtung.

Bei der Analytik von kontaminierten Oberflächen haben sich verschiedene Analysemethoden etabliert.

US 5,232,838 offenbart einen Träger aus einer selbsttragenden Stützschicht, auf welche eine wasserlösliche Klebeschicht aufgebracht ist. Auf die Klebeschicht ist ihrerseits ein wasserlösliches "Instant"-Pulver gestreut, welches bei Wasserkontakt ein Kulturmedium für Mikroorganismen bildet. Diese Schichtstruktur wird von einer abziehbaren Schutzfolie abgedeckt. Nach Abziehen der Schutzfolie kann die Klebeschicht mit dem "Instant"-Pulver auf eine kontaminierte Oberfläche angedrückt werden, um die Mikroben von der Oberfläche auf den Träger zu bringen. Anschließend wird das aufgeklebte "Instant"-Pulver mit Wasser in Kontakt gebracht, um die Kultivierung der Mikroorganismen durch das aus dem "Instant"-Pulver gebildete Nährmedium einzuleiten.

GB 2 019 434 A offenbart eine Haftklebefolie, bei welcher eine poröse Unterstützungsschicht aus Celluloseesterderivaten mit einer Klebeschicht aus beispielsweise Polyvinylpyrrolidon, Polyethylenglycol oder Polyvinylmethylether beschichtet ist. Die Unterstützungsschicht kann auf ihrer der Klebeschicht abgewandten Seite durch eine poröse abziehbare Schaumschicht mechanisch stabilisiert werden, die das Anpressen der Haftklebefolie auf eine mit Mikroben kontaminierte Oberfläche erleichtert. Nach Abnehmen der Haftklebefolie von der kontaminierten Oberfläche wird die Folie auf einem Nährmedium mit der der Klebeschicht abgewandten Seite abgelegt, um die aufgenommenen Mikroorganismen zu kultivieren.

EP 0 816 513 B1 offenbart Haftklebefolien, welche aus einer wasserdurchlässigen, aber mikrobenundurchlässigen Membran bestehen, auf welche eine Klebeschicht aus einem wasserlöslichen Polymer aufgebracht ist, wobei die Klebeschicht Mikroben fixieren kann. Die Membran kann optional auf eine Unterstützungsschicht aufgebracht sein. Mit Hilfe dieser Folien können Mikroorganismen mittels der Klebeschicht von kontaminierten Oberflächen entfernt werden. Die Folie, die in Form einer Filterronde vorliegen kann, kann dann in eine Filtrationseinheit eingebracht werden und mit einer wäßrigen Lösung, die eine anfärbende (chromogene) Substanz für die Mikroorganismen enthält, kontaktiert werden. Das wasserlösliche Polymer löst sich auf und passiert mit der wäßrigen Lösung die Membran, während die Mikroben auf der Membranoberfläche, auf der sich vormals die Klebeschicht befand, zurückgehalten werden, um nachfolgend analysiert zu werden.

Weit verbreitet ist außerdem der Einsatz von speziellen Wattestäbchen, sogenannten "Swabs". Hierbei handelt es sich um Stäbchen, die an einem Ende eine poröse Verdickung aufweisen, mit der die zu untersuchenden Oberflächen intensiv abgerieben werden und die dann in nachfolgenden Analyseschritten ausgewaschen werden, um die anhaftenden Kontaminationen zu untersuchen.
Diese Wattestäbchen werden für alle Arten von Oberflächen-Analysen eingesetzt, sei es für (bio)chemische Methoden, z. B. zur DNS-Analyse, oder auch für mikrobiologische Untersuchungen zur Bestimmung der Oberflächenverkeimung.

Zur quantitativen und semi-quantitativen Bestimmung von Oberflächenverkeimungen müssen anhaftende Mikroorganismen möglichst quantitativ vom Wattestäbchen gelöst werden, um die sich anschließenden weiteren Untersuchungsschritte nicht zu verfälschen. Nur im Fall einer Sterilkontrolle einer Oberfläche genügt die direkte Überführung in ein Nährmedium mit anschließender Wachstumsüberprüfung nach Inkubation.

Auf Grund des nie vollständig erfolgenden Überführungsschrittes ergibt sich, daß quantitative und semi-quantitative Analysen unter Verwendung derartiger Wattestäbchen schwierig zu validieren sind, weil zum einen Kontaminationen wie oben beschrieben nicht sicher quantitativ von den Wattestäbchen lösbar sind und zum anderen die Größe der Probefläche durch das Reiben mit "Swabs" nicht quantitativ definiert ist.

Daher sind für die quantitative Bestimmung von Oberflächenkontaminationen in erster Linie Kontakt-Agarplatten in Gebrauch, die nach entsprechender Bebrütung eine direkte Auswertung der Keimzahlen erlauben.

An Stelle der Swabs lassen sich auch Cellulosenitrat-Membranen zur quantitativen Bestimmung von Oberflächenkontaminationen einsetzen, da die elektrostatisch aufgeladene Membranoberfläche Keime von der zu untersuchenden Oberfläche abzieht und an der Membranoberflächenstruktur bindet. Diese Membranen können dann auf einen Agarnährboden überführt werden und analog der Methode unter Verwendung von Kontakt-Agarplatten nach Bebrütung quantitativ ausgewertet werden.

M. Pitzurra et al., Hygiene & Medizin, 22 (2) 1997, 77-92, mhp-Verlag, offenbaren diese Methode unter Verwendung von Cellulosenitratmembranen im Vergleich zu Methoden unter Verwendung von Kontakt-Agarplatten bzw. "Swabs" und zu einem Abwaschverfahren.
Die von M. Pitzurra et al. offenbarte Methode hat den Vorteil, daß die Oberflächen nicht wie beim Gebrauch von Kontakt-Agarplatten mit feuchtem Wachstums- oder Nährmedium verunreinigt werden und daß die Methode im Vergleich zu "Swabs" eine direkte quantitative Auswertung ermöglicht.
Zudem sind für den Bereich der filtrationsbasierten Keimzahlbestimmung für Flüssigkeiten mehrere Schnellmethoden verfügbar, so dass die Membranen nach den Probenahmen direkt diesen Methoden zugeführt werden können.

Trotz der genannten Vorteile gegenüber "Swabs" und Kontakt-Agarplatten hat sich diese von Pitzurra et al. untersuchte Methode bisher nicht etabliert, da die erforderliche sterile Handhabung der brüchigen Membranen, die typischerweise einen Durchmesser von ca. 50 mm und eine Dicke von 100-200 µm haben, sehr aufwendig und nicht verfahrensrobust ist. Damit erfordert die Probenahme einen hohen Zeitaufwand und Aufmerksamkeit bei der Entfernung der Membran von der zu analysierenden Oberfläche und bei der Weiterbehandlung der Membran. Außerdem erfordert dieses Verfahren die Manipulation der fragilen Membran mit weiteren technischen Hilfsmitteln, wie z. B. Pinzetten. Die Ergebnisse können durch Kontaminationen kompromittiert werden. Durch diese Nachteile ist insbesondere auch die reproduzierbare Probenahme, wie sie die GLP ("Good Laboratory Practice")- und die GMP ("Good Manufacturing Practice")-Vorschriften für die Pharmaindustrie fordern, erschwert.

WO 2008/113444 A1 offenbart eine Nährmedieneinheit zur Abnahme eines Filters von einer Filterunterstützungseinrichtung einer Filtrationsvorrichtung. Die Nährmedieneinheit umfaßt ein mit Nährmedium gefülltes Unterteil und einen Deckel.
Der Deckel hat einen in das Unterteil hineinragenden Fixierrand, der zur Entnahme des Filters aus der Filtrationsvorrichtung mit einem Rand des Filters über eine Klebehaftung mit dem Filter verbindbar ist. Mit Hilfe dieses Deckels ist es möglich, ohne Einsatz von Pinzetten oder anderer Hilfsmittel allein mit Hilfe des Fixierrands des Deckels den Filter von der Filterunterstützungseinrichtung in die Nährmedieneinheit zu überführen.
Die beschriebene Einheit eignet sich zur mikrobiologischen Untersuchung flüssiger Proben nach vorangegangener Filtration.

DE 20 2009 016 410 U1 offenbart eine Transfereinheit für die mikrobiologische Analyse zur Aufnahme eines porösen, scheibenförmigen Mediums, die das Medium mittels eines Fixierrandes aus einer ersten Behandlungsvorrichtung über den Rand des Mediums entnehmen kann und die eine mit einem abnehmbaren Deckel verschließbare Öffnung aufweist, über die eine Folgebehandlung des Mediums in einer weiteren Behandlungsvorrichtung durchführbar ist.

JP 2008/193919 A offenbart einen Kulturbehälter aus zwei über ein Scharniergelenk miteinander verbundenen buchdeckelförmigen Teilen zur Analyse von bakteriellen Kontaminationen auf Oberflächen. Der erste Teil, welcher als Deckel des Kulturbehälters fungiert, weist dabei eine kreisförmige Erhebung mit einer flächigen Klebstoff-Beschichtung auf. Der zweite Teil, welcher als Unterteil des Kulturbehälters fungiert, weist eine schalenförmige Vertiefung auf, in welche ein Nährmedium für Bakterien einfüllbar ist. Die klebstoffbeschichtete Erhebung des Deckels kann auf eine bakteriell kontaminierte Oberfläche aufgedrückt werden, wobei die Bakterien durch die Klebstoffbeschichtung auf der Erhebung fixiert werden. Anschließend wird der Deckel durch das Scharniergelenk auf das Unterteil geklappt und die auf der Erhebung fixierten Bakterien kontaktieren das Nährmedium im Unterteil über einen vollflächigen Kontakt zwischen der Erhebung und der Oberfläche des Nährmediums.

JP 2007/135542 A offenbart einen zu dem vorgenannten Kulturbehälter der JP 2008/193919 A ähnlichen Kulturbehälter, der einen separaten Deckel und ein separates Unterteil mit einem Nährmedium für zu analysierende Bakterien aufweist. Der vom Unterteil abnehmbare Deckel weist an seiner dem Nährmedium zugewandten Deckelinnenwand eine kreisförmige Klebstoffbeschichtung auf. Bakterien können mit Hilfe dieser Klebstoffbeschichtung von einer kontaminierten Oberfläche abgenommen werden und nach ihrer Fixierung auf der Klebstoffschicht wird der Deckel so auf das Unterteil gestülpt, daß die bakterienhaltige Klebstoffschicht des Deckels vollflächig auf der Nährmedienoberfläche im Unterteil klebt.

Die beiden vorgenannten Vorrichtungen, die sich prinzipiell bewährt haben, sind nicht geeignet, um eine Analyse von kontaminierten Oberflächen unter Verwendung von porösen scheibenförmigen Medien, wie z. B. Membranfiltern, durchzuführen, da in unerwünschter Weise die Analytik der zu untersuchenden Bakterien durch den die Bakterien fixierenden Klebstoff aus der Klebstoffbeschichtung gestört werden kann.

Aufgabe der vorliegenden Erfindung ist es, eine Vorrichtung und ein Verfahren bereitzustellen, bei welchen es möglich ist, eine kontaminierte Oberfläche mit mechanisch instabilen bzw. fragilen porösen scheibenförmigen Medien quantitativ zu analysieren, ohne kompliziert aufgebaute Systeme und ohne weitere technische Hilfsmittel einzusetzen und ohne daß ein Bruch oder eine Veränderung der zu analysierenden Oberfläche der scheibenförmigen Medien nach dem Entfernen von der kontaminierten Oberfläche und vor der Durchführung der Analyse auftritt.

Diese Aufgaben bezüglich der Vorrichtung und des Verfahrens werden durch die in den Ansprüchen gekennzeichneten Ausführungsformen der vorliegenden Erfindung gelöst.

Die die Vorrichtung betreffende Aufgabe wird durch die Vorrichtung nach Anspruch 1 gelöst, die eine Transfereinrichtung mit einem Träger und mit einem porösen scheibenförmigen Medium mit einer auf einer kontaminierten Oberfläche anordenbaren Kontaktseite umfaßt, wobei der Träger über einen ersten Fixierrand mit dem Medium auf dessen der Kontaktseite abgewandten Seite verbindbar ist, und die eine Analyseeinrichtung umfaßt, welche zur Abnahme des Mediums von dem Träger über einen zweiten Fixierrand mit dem Medium auf dessen Kontaktseite verbindbar ist, wobei der erste Fixierrand über eine erste Klebeverbindung und der zweite Fixierrand über eine zweite Klebeverbindung mit dem Medium verbindbar sind und wobei die erste Klebeverbindung durch eine geringere Kraftbeaufschlagung lösbar ist als die zweite Klebeverbindung.

Gemäß einer weiteren bevorzugten Ausführungsform ist zwischen dem Träger und dem Medium eine Unterstützungsschicht angeordnet, wobei die Analyseeinrichtung zur Abnahme des Mediums von der Unterstützungsschicht über den zweiten Fixierrand mit dem Medium auf dessen Kontaktseite verbindbar ist.
Dadurch, daß zwischen dem Träger und dem porösen scheibenförmigen Medium eine Unterstützungsschicht angeordnet ist, wird das fragile Medium durch die Unterstützungsschicht in der Transfereinrichtung vor Bruch und Oberflächenbeschädigung geschützt. Diese Anordnung des porösen Mediums in der Transfereinrichtung erlaubt es weiterhin, ohne Einwirkung von weiteren Hilfsmitteln auf das Medium (z. B. manuell oder mittels einer Pinzette) dieses mittels der Transfereinrichtung - analog einem Stempel - auf die kontaminierte Oberfläche zur Analyse aufzusetzen.

Dadurch, daß der erste Fixierrand über eine erste Klebeverbindung und der zweite Fixierrand über eine zweite Klebeverbindung mit dem Medium verbindbar sind und daß die erste Klebeverbindung durch eine geringere Kraftbeaufschlagung lösbar ist als die zweite Klebeverbindung, ergeben sich die nachfolgenden Vorteile in der Handhabung der erfindungsgemäßen Vorrichtung: Die Transfereinrichtung mit dem Medium kann stempelanalog mit dessen Kontaktseite auf die zu analysierende Oberfläche aufgebracht werden. Hierbei ist das poröse Medium über den ersten Fixierrand auf dem Träger bzw. auf der optional vorgesehenen Unterstützungsschicht der Transfereinrichtung fixiert. Nachfolgend wird die Transfereinrichtung der erfindungsgemäßen Vorrichtung von der Oberfläche abgehoben und umgedreht, so daß die Kontaktseite des Mediums, welche die Kontaminationen von der Oberfläche enthält, mit der Analyseeinrichtung der erfindungsgemäßen Vorrichtung verbunden werden kann. Ein zweiter Fixierrand der Analyseeinrichtung erlaubt nun das Ablösen des Mediums von dem Träger bzw. von der optional vorgesehenen Unterstützungsschicht der Transfereinrichtung und die Fixierung des Mediums an diesem zweiten Fixierrand, ohne daß hierfür weitere technische Hilfsmittel erforderlich sind. Die Ablösung des Mediums von der Transfereinrichtung mittels der Analyseeinrichtung wird dadurch möglich, daß zum Lösen der zweiten Klebeverbindung, die durch den zweiten Fixierrand vermittelt wird, eine höhere Kraft aufgewendet werden muß als für das Lösen der ersten Klebeverbindung, die durch den ersten Fixierrand vermittelt wird.

Im Sinne der vorliegenden Erfindungen sollen unter dem ersten Fixierrand, über welchen das Medium auf dessen der Kontaktseite abgewandten Seite mit dem Träger bzw. mit der optional vorgesehenen Unterstützungsschicht verbindbar ist, auch äquivalente Fixiereinrichtungen verstanden werden, wie z. B. eine flächige Klebeverbindung oder punktförmige Fixierungen, die eine Fixierung des Mediums auf dem Träger bzw. der Unterstützungsschicht erlauben, solange die durch diese flächigen oder punktförmigen Fixiereinrichtungen vermittelte Klebeverbindung durch eine geringere Kraftbeaufschlagung lösbar ist als die durch den zweiten Fixierrand vermittelte Klebeverbindung.

Die unterschiedlichen Stärken der beiden vorgenannten Klebeverbindungen, welche durch die unterschiedlichen, zum Lösen der Klebeverbindungen benötigten Kräfte bedingt sind, können bevorzugt dadurch eingestellt werden, daß beide Klebeverbindungen aus bezüglich ihrer physikalischen und/oder chemischen Eigenschaften verschiedenen Klebern gebildet sind, die auf das poröse scheibenförmigen Medium unterschiedliche Adhäsionskräfte ausüben.

Alternativ ist es denkbar, für beide Klebeverbindungen, die durch den ersten und zweiten Fixierrand vermittelt werden, denselben Kleber zu verwenden, aber den Grad der Bedeckung dieser Fixierränder mit Kleber für die beiden Klebeverbindungen zu variieren, wobei z. B. der zweite Fixierrand eine größere wirksame Klebefläche aufweist als der erste Fixierrand.

Außerdem können bei Einsatz desselben Klebers für den ersten und den zweiten Fixierrand die Kontaktseite und die von der Kontaktseite abgewandte Seite des porösen Mediums physikalisch oder chemisch unterschiedliche Oberflächeneigenschaften haben, so daß die der Kontaktseite abgewandte Seite des Mediums durch eine Klebeverbindung mit dem ersten Fixierrand fixierbar ist, die durch eine geringere Kraftbeaufschlagung lösbar ist als die zweite Klebeverbindung zwischen dem zweiten Fixierrand und der Kontaktseite des porösen Mediums.

Nach einer weiteren bevorzugten Ausführungsform sind die erste und die zweite Klebeverbindung temporär oder reversibel ausgebildet. Hierdurch wird zum einen das Ablösen des Mediums von dem Träger bzw. von der optional vorgesehenen Unterstützungsschicht der Transfereinrichtung durch die Analyseeinrichtung erleichtert und zum anderen ist es möglich, nachfolgend das von der Analyseeinrichtung fixierte Medium von dem zweiten Fixierrand zu entfernen und weiteren Analyseschritten in anderen Behandlungseinrichtungen zu unterziehen.

Der erste und der zweite Fixierrand weisen bevorzugt jeweils eine Haftschicht aus einem Kleber auf, der ein drucksensitiver Dispersionsklebstoff ist oder aus Acrylat-Copolymer-Mikrokugeln ausgebildet ist. Dadurch sind auch nasse oder feuchte poröse Medien leicht an dem ersten Fixierrand des Trägers bzw. der Unterstützungsschicht der Transfereinrichtung bzw. an dem zweiten Fixierrand der Analyseeinrichtung fixierbar bzw. für Folgebehandlungen von dem zweiten Fixierrand abziehbar.

Die Haftschichten können entweder an dem ersten und zweiten Fixierrand der Transfereinrichtung und der Analyseeinrichtung oder an den zu diesen Fixierrändern jeweils korrespondierenden Rändern des porösen Mediums aufgebracht sein.

Nach einer weiteren Ausführungsform der Erfindung sind die Kleber sterilisierbar. Die Kleber sind bevorzugt DNA- und proteinfrei und zeigen keine unspezifischen Reaktionen mit Reagenzien, die für die Schnellanalytik mikrobieller Kontaminationen verwendet werden (z. B. antikörperbasierte Reagenzien oder Reagenzien, die für eine Polymerase-Kettenreaktion (PCR-Reaktion) verwendet werden). Insbesondere können Kleber verwendet werden, die nur geringe Autofluoreszenzen aufweisen und nicht unspezifisch mit entsprechenden Färbe- und Markierungsreagenzien für die Analyse der kontaminierten Oberfläche reagieren. Dies gilt insbesondere für in der Auswertung gängige Wellenlängenbereiche von 400 bis 800 nm. Auch können Kleber verwendet werden, die keine antibiotischen, antiviralen oder fungiziden Eigenschaften aufweisen.

Bevorzugt ist der Kleber über eine wasserlösliche Zwischenschicht an den ersten und/oder zweiten Fixierrand aufgebracht. Für die Untersuchung nasser bzw. feuchter kontaminierter Oberflächen oder für den Fall, daß das poröse Medium vor Probenahme mit steriler Flüssigkeit zur verbesserten Probennahmeeffizienz vorbenetzt wird, ist besonders bevorzugt an den ersten Fixierrand der Transfereinrichtung zwischen diesem Fixierrand und dem Kleber die wasserlösliche Zwischenschicht angebracht, während der zweite Fixierrand der Analyseeinrichtung keine derartige Zwischenschicht aufweist. Bei dieser Ausführungsform löst sich die wasserlösliche Zwischenschicht am ersten Fixierrand nach längerem Kontakt des feuchten, porösen Mediums allmählich auf, wobei die durch den ersten Fixierrand vermittelte Klebkraft gegen Null sinkt. Anschließend ist durch die Klebeverbindung mit dem zweiten Fixierrand der Analyseeinrichtung das poröse Medium leicht von dem Träger bzw. der optional vorgesehenen Unterstützungsschicht der umgedrehten Transfereinrichtung abhebbar.

Alternativ weist nur der zweite Fixierrand, nicht aber der erste Fixierrand eine wasserlösliche Zwischenschicht auf. Diese Ausführungsform hat den Vorteil, daß das poröse Medium nach seiner Entfernung aus der Transfereinrichtung und nach seiner Fixierung an dem zweiten Fixierrand der Analyseeinrichtung mit einer Flüssigkeit benetzt werden kann, die zeitversetzt die wasserlösliche Zwischenschicht auflöst, so daß das poröse Medium für Folgebehandlungen leicht von dem zweiten Fixierrand der Analyseeinrichtung abgezogen werden kann.

Bei einer bevorzugten Ausführungsform ist die Unterstützungsschicht der Transfereinrichtung porös und besteht aus einem elastischen Kunststoffschaum. Hierdurch wird eine gleichmäßige Druckbeaufschlagung der zu analysierenden Oberfläche mittels des porösen Mediums sichergestellt. Besonders bevorzugt besteht die Unterstützungsschicht aus einem Material, welches bei der Entfernung des porösen scheibenförmigen Mediums von der Transfereinrichtung durch die Analyseeinrichtung keine Rückstände auf der der Kontaktseite des Mediums abgewandten Seite hinterläßt.
Die Verwendung eines elastischen Kunststoffschaumes als Unterstützungsschicht hat den weiteren Vorteil, daß gemäß der offenen, porösen Struktur nur geringe Kontaktflächen zum Medium vorliegen und damit automatisch geringere Klebekräfte vorliegen im Vergleich zu glatten, nicht porösen Unterstützungsschichten. Hierdurch kann automatisch sichergestellt werden, daß die erste Klebeverbindung durch eine geringere Kraftbeaufschlagung lösbar ist als die zweite Klebeverbindung zwischen dem zweiten Fixierrand und der Kontaktseite des Mediums.

Die Unterstützungsschicht verbindet nicht nur den Träger mit dem porösen Medium, sondern optimiert auch durch ihre Flexibilität und Tiefe den Kontakt und ermöglicht einen gleichförmigen Anpreßdruck auf die kontaminierte Oberfläche über die Kontaktseite des porösen Mediums.
Die Unterstützungsschicht ist bevorzugt fest mit dem Träger verbunden und weist dieselbe Flächengeometrie wie das poröse Medium auf.
Die Unterstützungsschicht kann in der Höhe ihrer Dicke von dem z. B. wannenförmigen Träger umschlossen sein, so dass sich ein Trog ergibt, der von der Unterstützungsschicht ausgefüllt ist.

Nach einer weiteren bevorzugten Ausführungsform ist die Transfereinrichtung als Bestandteil eines Nährmedienbehälters ausgebildet. Dabei ist die Unterstützungsschicht der Transfereinrichtung an der Unterseite des Nährmedienbehälters über eine irreversible oder reversible Haftverbindung angebracht. Auf der Oberseite des Nährmedienbehälters befindet sich das Nährmedium, z. B. ein Nährboden. Der Transfer des porösen Mediums von der Unterstützungsschicht an der Unterseite des Nährmedienbehälters auf die Analyseeinrichtung erfolgt bei dieser Ausführungsform durch die Transfereinrichtung, wobei die erste Klebeverbindung, wie vorstehend beschrieben, durch eine geringere Kraftbeaufschlagung lösbar ist als die zweite Klebeverbindung. Die für den Transfer verwendbare Analyseeinrichtung kann dergestalt ausgebildet sein, daß sie gleichzeitig als Deckel für den Nährmedienbehälter dient.

Besonders bevorzugt ist die Unterstützungsschicht mit einer Analyseflüssigkeit oder mit einer Behandlungsflüssigkeit für eine Folgebehandlung des porösen Mediums getränkt. Diese Ausführungsform hat sich besonders bewährt, wenn der Träger als Wanne für die poröse Unterstützungsschicht ausgebildet ist, so daß er mit den vorgenannten Flüssigkeiten gefüllt werden kann und damit z. B. eine direkte Bebrütung von auf das poröse Medium überführten Keimen nach der Flüssigkeitszufuhr möglich wird. Geeignete Analyse- und Behandlungsflüssigkeiten sind in der Mikrobiologie bekannt.

Gemäß einer weiteren bevorzugten Ausführungsform ist das scheibenförmige poröse Medium ein Membranfilter. Bei Verwendung einer mit Analyse- bzw. Behandlungsflüssigkeit (z. B. Nährmedien) getränkten porösen Unterstützungsschicht sollte die Unterstützungsschicht und ihre Verbindung zum Membranfilter über den ersten Fixierrand für die Direktauswertung der Keime eine optimierte Diffusion und Versorgung mit den Nährmedien gewährleisten. Für diese Anwendung ist es von Vorteil, wenn die Fläche des Membranfilters im Durchmesser etwas kleiner dimensioniert ist als die Fläche der Unterstützungsschicht, so daß beim Tränken der Unterstützungsschicht mit dem Nährmedium die verdrängte Luft seitlich austreten kann.

Die Transfereinheit der erfindungsgemäßen Vorrichtung weist bevorzugt eine zylindrische Geometrie auf, ist aber nicht auf diese Geometrie beschränkt, da weiterführende korrespondierende Einheiten zur Folgebehandlung andere Geometrien erfordern können.

Bei einer besonders bevorzugten Ausführungsform der erfindungsgemäßen Vorrichtung wird der zweite Fixierrand der Analyseeinrichtung von einer freien Stirnfläche einer ringförmigen Wandung gebildet, welche an einer Deckelinnenfläche der Analyseeinrichtung angeordnet ist.
Durch diese Ausführung ist das poröse Medium nach erfolgter Oberflächenanalyse durch den zweiten Fixierrand der Analyseeinrichtung von dem Träger bzw. von der optional zwischen Träger und Medium vorgesehenen Unterstützungsschicht der Transfereinrichtung abhebbar, weil die von dem zweiten Fixierrand vermittelte Haftkraft die von dem ersten Fixierrand der Transfereinrichtung auf das Medium ausgeübte Haftkraft übersteigt.

Die Analyseeinrichtung ist in ihrer umlaufenden Kontur funktional auf die Kontur bzw. Struktur der Transfereinrichtung so abgestimmt, dass der zweite Fixierrand mit dem Medium nur in dessen peripheren, ringförmigen Bereich durch eine Klebeverbindung verbindbar ist. Dieser periphere Bereich ist nicht zur weiteren Analyse verwendbar. Somit gibt bei dieser Ausführungsform der innere Durchmesser des zweiten Fixierrands der Analyseeinrichtung das wirksame, einer Analyse zugängliche Oberflächenareal des porösen Mediums vor. Kontaminationen in diesem Randbereich werden bei der Analyse nicht berücksichtigt.

Gemäß einer alternativen bevorzugten Ausführungsform weist die Analyseeinrichtung eine mit einem abnehmbaren Deckel verschließbare Öffnung auf, über die eine Folgebehandlung des porösen Mediums durchführbar ist. Durch diese verschließbare Öffnung ist es möglich, die die Kontaminationen enthaltende Kontaktseite des porösen Mediums weiter zu analysieren oder weitere Behandlungseinrichtungen, die in diese Öffnung einsetzbar sind, mit der Kontaktseite in Kontakt zu bringen.

Die weitere Aufgabe bezüglich des Verfahrens zur Analyse einer kontaminierten Oberfläche wird durch das Verfahren nach Anspruch 11 gelöst, welches die folgenden Schritte umfaßt:
A) Anordnen der Kontaktseite des porösen scheibenförmigen Mediums der Transfereinrichtung auf der kontaminierten Oberfläche, wobei das Medium auf seiner der Kontaktseite abgewandten Seite über den ersten Fixierrand mit dem Träger der Transfereinrichtung verbunden ist,
B) Aufnehmen von Kontaminationen der kontaminierten Oberfläche in das Medium über die Kontaktseite,
C) Entfernen der Transfereinrichtung mit dem die Kontaminationen enthaltenden Medium von der kontaminierten Oberfläche,
D) Verbinden der Kontaktseite des die Kontaminationen enthaltenden Mediums in der Transfereinrichtung über den zweiten Fixierrand mit der Analyseeinrichtung,
E) Ablösen des nach Schritt D) über die Kontaktseite mit der Analyseeinrichtung verbundenen Mediums von dem Träger der Transfereinrichtung und
F) Analysieren des die Kontaminationen enthaltenden Mediums, welches mit der Analysevorrichtung verbunden ist,
wobei das Ablösen in Schritt E) durch eine durch den zweiten Fixierrand vermittelte Klebeverbindung ausgelöst wird, die eine größere Haftkraft auf das Medium ausübt als eine durch den ersten Fixierrand vermittelte Klebeverbindung.

Dadurch, daß das Ablösen in Schritt E) durch eine durch den zweiten Fixierrand vermittelte Klebeverbindung ausgelöst wird, die eine größere Haftkraft auf das Medium ausübt als eine durch den ersten Fixierrand vermittelte Klebeverbindung, ist es möglich, das poröse Medium nach erfolgter Analyse der kontaminierten Oberfläche von dem ersten Fixierrand der Transfereinrichtung abzuziehen und gleichzeitig an dem zweiten Fixierrand der Analyseeinrichtung zu fixieren, ohne daß für diesen Transfer des porösen Mediums von der kontaminierten Oberfläche in die Analyseeinrichtung zusätzliche technische Hilfsmittel (wie z. B. Pinzetten, Finger) erforderlich sind.

Nach einer alternativen Ausführungsform des Verfahrens ist zwischen dem Träger und dem Medium eine poröse Unterstützungsschicht angeordnet, welche vor Schritt A) oder nach Schritt B) mit einer Analyseflüssigkeit oder mit einer Behandlungsflüssigkeit für eine Folgebehandlung des Mediums getränkt wird.

Besonders bevorzugt wird für das erfindungsgemäße Verfahren als poröses Medium ein Membranfilter verwendet. Bei den Kontaminationen handelt es sich vorzugsweise um Mikroben, wie Bakterien oder Pilze. Besonders bevorzugt ist der Membranfilter eine die vorgenannten Mikroben zurückhaltende mikroporöse Membran.

Bei einer weiteren bevorzugten Ausführungsform des Verfahrens erfolgt in Schritt F) zusätzlich das Aufsetzen des über den zweiten Fixierrand mit der Analyseeinrichtung verbundenen porösen Mediums auf die Oberfläche eines Nährbodens im Unterteil eines Nährmedienbehälters.
Die Analyseeinrichtung mit dem an dem zweiten Fixierrand fixierten Medium kann so auf der Oberfläche eines in dem Unterteil der Nährmedieneinheit (z. B. Petrischale) angeordneten Nährbodens (z. B. Agar) zur Bebrütung abgelegt werden, wobei das Unterteil der Nährmedieneinheit durch die als Deckel fungierende Analyseeinrichtung abgedeckt wird.

Eine weitere bevorzugte Ausführungsform kombiniert die Funktion der Transfereinrichtung mit der eines Unterteils eines Nährmedienbehälters und die Funktion der Analyseeinrichtung als Deckel des Nährmedienbehälters in der Weise, daß die Transfereinrichtung auf der dem porösen Medium entgegengesetzten Seite als Bestandteil des Unterteils des Nährmedienbehälters ausgebildet ist.
Dabei ist die Unterstützungsschicht der Transfereinrichtung an der Unterseite des Nährmedienbehälters über eine wahlweise irreversible oder reversible Haftverbindung angebracht. Auf der Oberseite des Nährmedienbehälters befindet sich das Nährmedium, z. B. ein Nährboden. Der Transfer des porösen Mediums von der Unterstützungsschicht an der Unterseite des Nährmedienbehälters auf die Analyseeinrichtung erfolgt bei dieser Ausführungsform dadurch, daß mittels des zweiten Fixierrands der Analyseeinrichtung das poröse Medium von der Unterstützungsschicht abgelöst wird und an dem zweiten Fixierrand fixiert wird, wobei nachfolgend das poröse Medium auf der Oberseite des Nährmediums zu liegen kommt und die Analyseeinrichtung als Deckel des Nährmedienbehälters das Nährmedium abdeckt.

Weitere Merkmale der Erfindung ergeben sich aus der nachfolgenden ausführlichen Beschreibung und den beigefügten Zeichnungen, in denen bevorzugte Ausführungsformen der Erfindung beispielhaft veranschaulicht sind.

In den Figuren zeigen:
- Figur 1:: eine Seitenansicht einer Transfereinrichtung der erfindungsgemäßen Vorrichtung im Schnitt,
- Figur 2:: eine Seitenansicht der erfindungsgemäßen Vorrichtung mit der Transfereinrichtung von Figur 1 und mit einer auf die Transfereinrichtung aufgesetzten Analyseeinrichtung im Schnitt,
- Figur 3:: eine Seitenansicht einer weiteren Ausführungsform der Transfereinrichtung der erfindungsgemäßen Vorrichtung im Schnitt und
- Figur 4:: eine Seitenansicht einer weiteren Ausführungsform der erfindungsgemäßen Vorrichtung mit der Transfereinrichtung gemäß Figur 1, die als Bestandteil eines Unterteils eines Nährmedienbehälters ausgebildet ist, wobei die Analyseeinrichtung den Deckel des Nährmedienbehälters bildet.

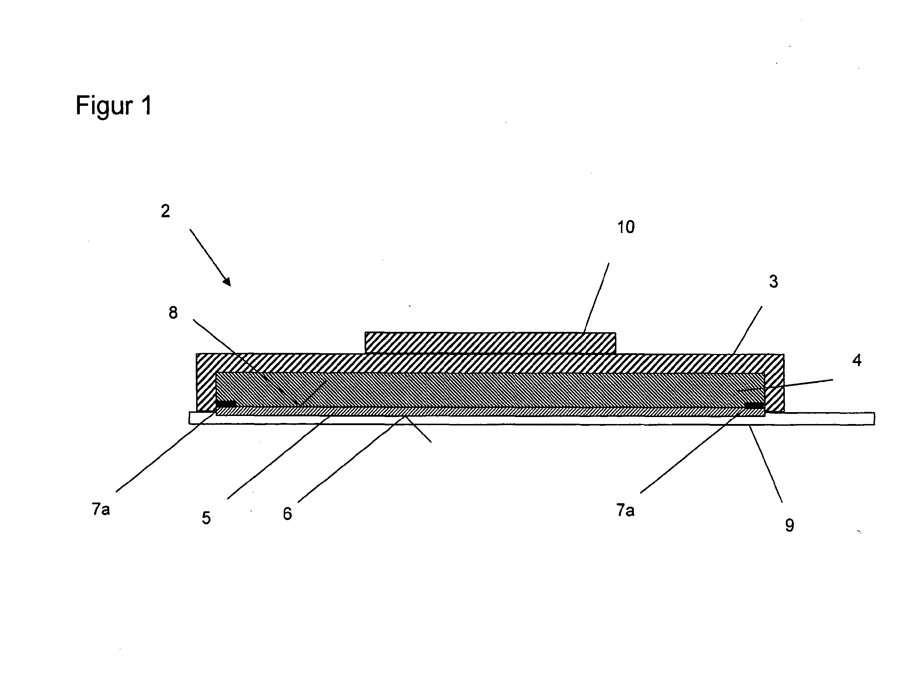

Gemäß Figur 1 umfaßt die erfindungsgemäße Vorrichtung **1** eine Transfereinrichtung **2** mit einem Träger **3,** welcher besonders bevorzugt als Trog ausgebildet ist, in welchem die Unterstützungsschicht **4** angeordnet ist. Die Transfereinrichtung **2** weist weiterhin ein poröses, scheibenförmiges Medium **5** mit einer Kontaktseite **6** auf. Die Unterstützungsschicht **4** ist über einen ersten Fixierrand **7a** mit der der Kontaktseite **6** abgewandten Seite **8** des Mediums **5** verbindbar. Bei dem ersten Fixierrand **7a** handelt es sich um eine Klebeverbindung, die sicherstellt, daß das Medium **5** in seinem Randbereich an der Unterstützungsschicht **4** haften bleibt. Die Unterstützungsschicht **4** stützt das poröse Medium **5** vollflächig auf dessen der Kontaktseite **6** abgewandten Seite **8** ab und bewahrt es vor mechanischer Beschädigung. Die Unterstützungsschicht **4** kann porös sein und eine Analyse- oder Behandlungsflüssigkeit enthalten, mit welcher das poröse Medium **5** vor oder während der Analyse benetzbar ist.

Als optionales Merkmal kann die Transfereinrichtung **2** eine sterile Abdeckung **9** in Form einer Abziehfolie mit Lasche aufweisen, welche die Kontaktseite **6** des porösen Mediums **5** vor Beschädigung und unerwünschter Kontamination schützt, bevor eine Analyse einer kontaminierten Oberfläche durchgeführt wird. Weiterhin kann die Transfereinrichtung optional einen Griff **10** am Träger **3** aufweisen. Der Griff **10** erleichtert die stempelanaloge Handhabung der Transfereinrichtung **2.**

Unter dem Fixierrand **7a** sollen hierbei auch äquivalente Fixiereinrichtungen verstanden werden, die von einer randförmigen Ausführungsform abweichen und die eine vollflächige oder punktförmige Fixierung des Mediums **5** an der Unterseite der Unterstützungsschicht **4** erlauben.
Die vollflächige Ausgestaltung des Fixierrandes **7a** über die gesamte Unterseite der Unterstützungsschicht **4** bietet sich insbesondere dann an, wenn als Unterstützungsschicht **4** ein poröser Polymerschaum verwendet wird. Auf Grund der geringen Kontaktpunkte der Stege des Polymerschaums zum Medium **5** kann selbst eine über die gesamte Unterseite der Unterstützungsschicht **4** ausgeführte Klebeverbindung im Sinne eines Fixierrands **7a** eine geringere Haftkraft vermitteln als die Haftkraft, die von dem zweiten, vorzugsweise ringförmig ausgebildeten Fixierrand **7b** vermittelt wird.

Zur Durchführung einer Analyse einer kontaminierten Oberfläche wird ggf. zunächst die sterile Abdeckung **9** von der Kontaktseite **6** entfernt. Mit Hilfe des optionalen Griffes **10** kann nun die Transfereinrichtung **2** mit der exponierten Kontaktseite **6** wie ein Stempel auf die zu analysierende kontaminierte Oberfläche aufgesetzt werden, wobei die Kontaktseite **6** des porösen Mediums **5** vollflächig auf der kontaminierten Oberfläche zu liegen kommt und die Kontaminationen (z. B. Mikroben) über die Kontaktseite **6** von dem porösen Medium **5** aufgenommen werden.

Nach erfolgter Einwirkung der Transfereinrichtung **2** auf die zu analysierende Oberfläche wird die Transfereinrichtung **2** von der Oberfläche abgehoben und umgedreht.

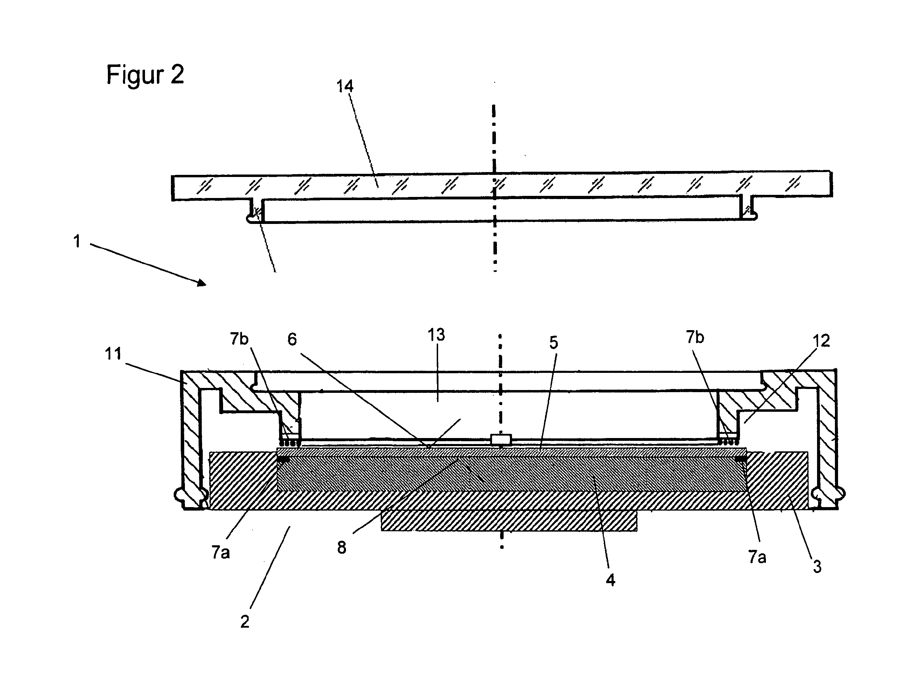

Die Transfereinrichtung wird gemäß Figur 2 nach dem Abheben und Umdrehen so mit der Analyseeinrichtung **11** verbunden, daß der Rand der Kontaktseite **6** des Mediums **5** auf dem zweiten Fixierrand **7b** der Analyseeinrichtung **11** zu liegen kommt. Dieser zweite Fixierrand **7b** ist wie der erste Fixierrand **7a** als Klebeverbindung ausgebildet. Diese Klebeverbindung durch den zweiten Fixierrand **7b** ist im Vergleich zu der Klebeverbindung durch den ersten Fixierrands **7a** aber so ausgebildet, daß der zweite Fixierrand **7b** auf die Kontaktseite **6** des Mediums **5** eine größere Haftkraft ausübt als der erste Fixierrand **7a** auf die der Kontaktseite **6** abgewandte Seite **8** des Mediums 5. Diese Varianz in den beiden Haftkräften kann z. B. - wie in Figur 2 exemplarisch angedeutet - durch die Breite der die Klebeschicht aufweisenden Fixierränder eingestellt werden, wobei für beide Klebeschichten der gleiche Kleber verwendet wird. Der Fixierrand **7b** ist dabei breiter ausgeführt als der Fixierrand **7a.**

Alternativ ist diese Varianz der von dem ersten und zweiten Fixierrand ausgeübten Haftkräfte auch dadurch möglich, daß für beide Fixierränder jeweils der gleiche Kleber in gleicher Breite der Klebschicht verwendet wird, sich aber die Oberflächeneigenschaften der Kontaktseite **6** und der von dieser abgewandten Seite **8** des porösen Mediums **5** physikalisch oder chemisch so voneinander unterscheiden, daß beide Seiten **6** und **8** unterschiedlich stark an den Fixierrändern **7b** bzw. **7a** haften.

Bei einer weiteren Ausführungsform der erfindungsgemäßen Vorrichtung **1** werden jeweils chemisch oder physikalisch verschiedene Kleber als Haftschicht für den ersten und zweiten Fixierrand **7a** und **7b** verwendet, die unterschiedlich hohe Haftkräfte auf die Kontaktseite **8** und die von dieser abgewandte Seite **6** ausüben.

Dadurch, daß der zweite Fixierrand **7b** eine stärkere Haftkraft auf das poröse Medium **5** ausübt als der erste Fixierrand **7a,** kann das Medium **5** ohne Zuhilfenahme weiterer Hilfsmittel mit Hilfe des Fixierrands **7b** der Analyseeinrichtung **11** von der Unterstützungsschicht **4** der Transfereinrichtung **2** abgelöst werden.

Bei der Ausführungsform nach Figur 2 wird der zweite Fixierrand **7b** von einer freien Stirnfläche einer ringförmigen Wandung **12** gebildet.

Während des Ablösevorgangs kann die Transfereinrichtung **2** durch eine um den Träger **3** herum verlaufende, nicht gezeigte Schnapp-, Klemm-, Einrast- oder Bajonettverbindung mit der Analyseeinrichtung **11** reversibel verbunden werden.

Nachdem das poröse Medium **5** über den zweiten Fixierrand **7b** auf seiner Kontaktseite **6** mit der Analyseeinrichtung **11** verbunden ist, kann die Analyseeinrichtung **11** von der Transfereinrichtung **2** getrennt werden. Gemäß Figur 2 kann die Analyseeinrichtung **11** eine mit einem Deckel **14** verschließbare Öffnung **13** aufweisen. Hierdurch ist es möglich, auf der der Öffnung **13** zugewandten Kontaktseite 6 des Mediums 5 Folgebehandlungen durchzuführen.

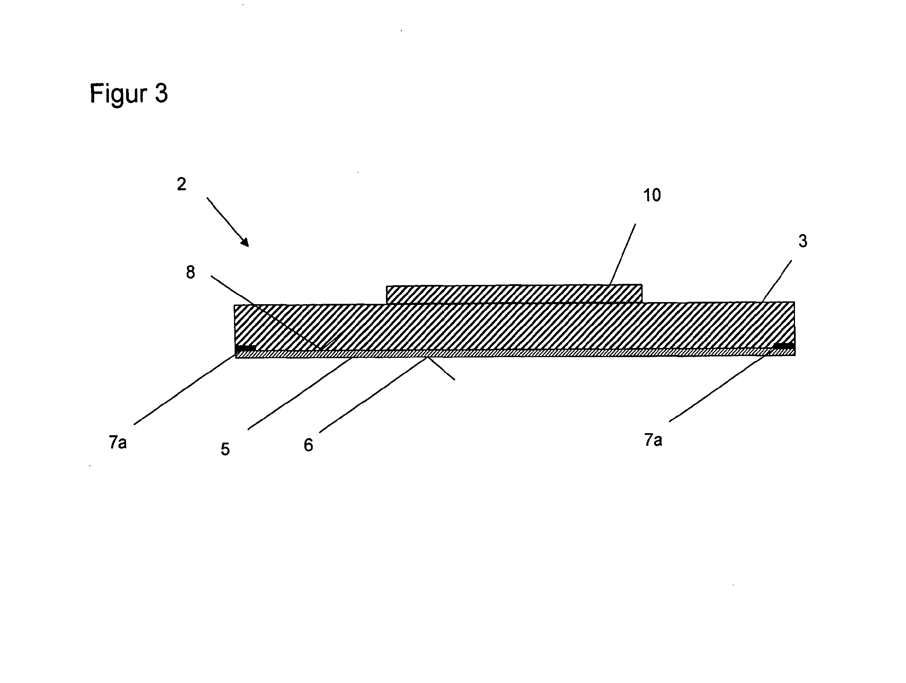

Alternativ ist gemäß der in Figur 3 gezeigten Ausführungsform der Transfereinheit **2** denkbar, daß auf die in Figur 2 dargestellte Unterstützungsschicht **4** verzichtet wird und daß bei dieser Ausführungsform das Medium **5** direkt und unmittelbar über den Fixierrand **7a** vollflächig mit der dem Griff **10** abgewandten Unterseite des Trägers **3** verbindbar ist, wobei das Medium **5** mechanisch durch die Unterseite des Trägers **3** unterstützt wird.

Bei der in Figur 3 dargestellten Ausführungsform der Transfereinheit **2** wirkt der Träger **3** gleichzeitig als Unterstützungsschicht für das Medium **5.** Die Transfereinrichtung **3** weist ein poröses, scheibenförmiges Medium **5** mit einer Kontaktseite **6** auf. Der Träger **3** ist über einen ersten Fixierrand **7a** direkt und unmittelbar mit der der Kontaktseite **6** abgewandten Seite **8** des Mediums **5** verbindbar. Bei dem ersten Fixierrand **7a** handelt es sich um eine Klebeverbindung, die sicherstellt, daß das Medium **5** in seinem Randbereich an der Unterseite des Trägers **3** wenigstens für die Dauer des Kontaktes des Mediums **5** mit der kontaminierten Oberfläche haften bleibt. Der Träger **3** stützt das poröse Medium **5** vollflächig auf dessen der Kontaktseite **6** abgewandten Seite **8** ab und bewahrt es vor mechanischer Beschädigung. Handhabungshilfen, wie ein optionaler Griff **10,** erleichtern die stempelanaloge Handhabung der Transfereinrichtung **2.**

Auch bei der Ausführungsform nach Figur 3 sollen erfindungsgemäß unter dem Fixierrand **7a** äquivalente Fixiereinrichtungen verstanden werden, die von einer randförmigen Ausführungsform abweichen und die eine vollflächige oder punktförmige Fixierung des Mediums **5** an der Unterseite des Trägers **3** erlauben.

Die vollflächige Ausgestaltung des Fixierrandes **7a** über die gesamte, dem Griff **10** abgewandte Unterseite des Trägers **3** bietet sich insbesondere dann an, wenn als Träger **3** ein poröser, mechanisch stabiler, bevorzugt nicht kompressibler Polymerschaum verwendet wird. Auf Grund der geringen Kontaktpunkte der Stege des Polymerschaums zum Medium **5** kann selbst eine über die gesamte Unterseite des Trägers **3** ausgeführte Klebeverbindung im Sinne eines Fixierrands **7a** eine geringere Haftkraft vermitteln als die Haftkraft, die von dem zweiten, vorzugsweise ringförmig ausgebildeten Fixierrand **7b** vermittelt wird.

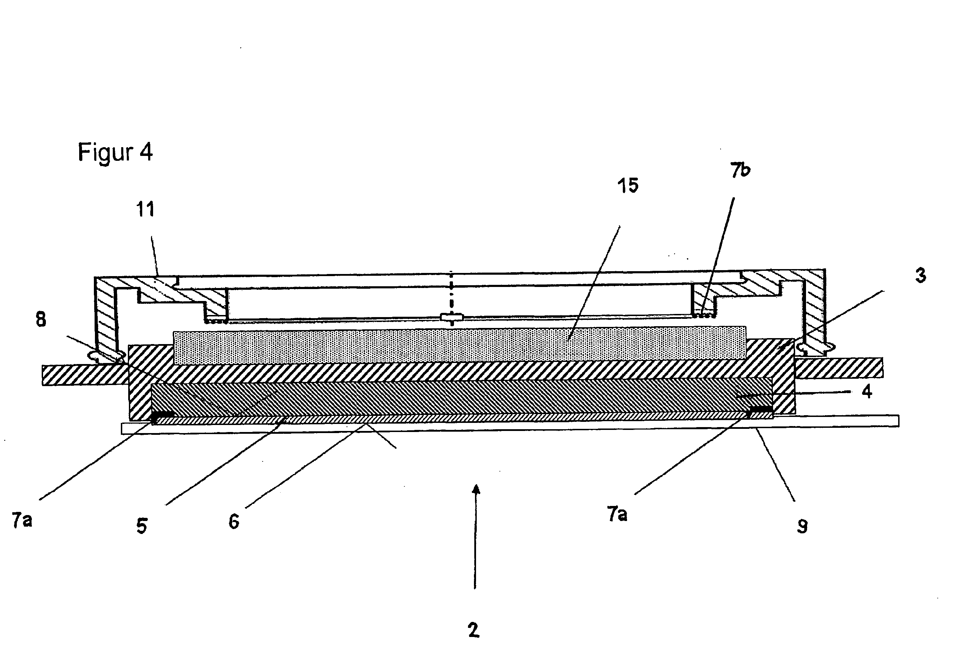

Bei einer weiteren Ausführungsform gemäß der Figur 4 ist es möglich, die Analyseeinrichtung **11** als Deckel für ein Unterteil eines Nährmedienbehälters mit einem Nährboden **15** zu verwenden. Das Unterteil weist hierbei bevorzugt eine nicht gezeigte Innenwandung auf, welche mit dem zweiten Fixierrand **7b** der Analyseeinrichtung **11** korrespondiert.
Die Analyseeinrichtung **11** kann mit dem am zweiten Fixierrand **7b** fixierten Medium **5** auf das Unterteil des Nährmedienbehälters aufgesetzt werden, wobei die der Kontaktseite **6** abgewandte Seite **8** des Mediums **5** vollflächig auf der Oberfläche des Nährbodens zu liegen kommt. In Figur 4 ist ein für eine Oberflächenanalyse einsatzbereiter Nährmedienbehälter dargestellt, bei welchem das poröse Medium **5** auf der dem Nährboden **15** abgewandten Seite auf der Unterstützungsschicht **4,** welche Teil des Unterteils des Nährmedienbehälters ist, fixiert ist und durch eine sterile Abdeckung **9** geschützt ist.

Gemäß dieser Ausführungsform der Figur 4 ist die Transfereinrichtung **2** als Bestandteil des Unterteils eines Nährmedienbehälters ausgebildet. Die Oberseite des Unterteils des Nährmedienbehälters weist hierbei eine Aufnahmeeinrichtung für den Nährboden **15** auf. Die Analyseeinrichtung **11** fungiert in dieser Ausführungsform gleichzeitig als Deckel des Nährmedienbehälters. Der Träger **3** der Transfereinrichtung **2** ist hierbei gleichzeitig der Behälter für den Nährboden **15,** der von der Analyseeinrichtung **11** als Oberteil des Nährmedienbehälters abgedeckt wird und mit der Analyseeinrichtung **11** in den Abmessungen korrespondiert.

## Patentansprüche

1. Vorrichtung **(1)** zur Analyse einer kontaminierten Oberfläche,
umfassend
- eine Transfereinrichtung **(2)**
mit einem Träger **(3)** und
mit einem porösen scheibenförmigen Medium **(5)** mit einer auf der kontaminierten Oberfläche anordenbaren Kontaktseite **(6),**
wobei der Träger **(3)** über einen ersten Fixierrand **(7a)** mit dem Medium **(5)** auf dessen der Kontaktseite **(6)** abgewandten Seite **(8)** verbindbar ist, und
- eine Analyseeinrichtung **(11),**
die zur Abnahme des Mediums **(5)** von dem Träger **(3)** über einen zweiten Fixierrand **(7b)** mit dem Medium **(5)** auf dessen Kontaktseite **(6)** verbindbar ist,
wobei der erste Fixierrand **(7a)** über eine erste Klebeverbindung und der zweite Fixierrand **(7b)** über eine zweite Klebeverbindung mit dem Medium **(5)** verbindbar sind und wobei die erste Klebeverbindung durch eine geringere Kraftbeaufschlagung lösbar ist als die zweite Klebeverbindung.

2. Vorrichtung **(1)** nach Anspruch 1,
wobei zwischen dem Träger **(3)** und dem Medium **(5)** eine Unterstützungsschicht **(4)** angeordnet ist und wobei die Analyseeinrichtung **(11)** zur Abnahme des Mediums **(5)** von der Unterstützungsschicht **(4)** über den zweiten Fixierrand **(7b)** mit dem Medium **(5)** auf dessen Kontaktseite **(6)** verbindbar ist.

3. Vorrichtung **(1)** nach einem der vorhergehenden Ansprüche,
wobei die erste und die zweite Klebeverbindung temporär oder reversibel ausgebildet sind.

4. Vorrichtung **(1)** nach einem der vorhergehenden Ansprüche,
wobei der erste und zweite Fixierrand **(7a, 7b)** jeweils eine Haftschicht aus einem Kleber aufweisen, der ein drucksensitiver Dispersionsklebstoff ist oder aus Acrylat-Copolymer-Mikrokugeln ausgebildet ist.

5. Vorrichtung **(1)** nach Anspruch 4,
wobei der Kleber über eine wasserlösliche Zwischenschicht an den ersten und/oder zweiten Fixierrand **(7a, 7b)** aufgebracht ist.

6. Vorrichtung **(1)** nach einem der vorhergehenden Ansprüche 2-5,
wobei die Unterstützungsschicht **(4)** porös ist und aus einem elastischen Kunststoffschaum besteht.

7. Vorrichtung **(1)** nach Anspruch 6,
wobei die Unterstützungsschicht **(4)** mit einer Analyseflüssigkeit oder mit einer Behandlungsflüssigkeit für eine Folgebehandlung des porösen Mediums **(5)** getränkt ist.

8. Vorrichtung **(1)** nach einem der vorhergehenden Ansprüche,
wobei der zweite Fixierrand **(7b)** von einer freien Stirnfläche einer ringförmigen Wandung **(12)** gebildet wird, welche an einer Deckelinnenfläche der Analyseeinrichtung **(11)** angeordnet ist.

9. Vorrichtung **(1)** nach einem der vorhergehenden Ansprüche,
wobei die Analyseeinrichtung **(11)** eine mit einem abnehmbaren Deckel **(14)** verschließbare Öffnung **(13)** aufweist, über die eine Folgebehandlung des Mediums **(5)** durchführbar ist.

10. Vorrichtung **(1)** nach einem der vorhergehenden Ansprüche 2 bis 9, wobei die Transfereinrichtung **(2)** als Bestandteil eines Nährmedienbehälters ausgebildet ist, wobei die Unterstützungsschicht **(4)** der Transfereinrichtung **(2)** an der Unterseite des Nährmedienbehälters über eine irreversible oder reversible Haftverbindung angebracht ist.

11. Verfahren zur Analyse einer kontaminierten Oberfläche unter Verwendung einer Vorrichtung **(1)** nach einem der Ansprüche 1 bis 10,
umfassend die Schritte:
A) Anordnen der Kontaktseite **(6)** des porösen scheibenförmigen Mediums **(5)** der Transfereinrichtung **(2)** auf der kontaminierten Oberfläche, wobei das Medium **(5)** auf seiner der Kontaktseite **(6)** abgewandten Seite **(8)** über den ersten Fixierrand **(7a)** mit dem Träger **(3)** der Transfereinrichtung **(2)** verbunden ist,
B) Aufnehmen von Kontaminationen der kontaminierten Oberfläche in das Medium **(5)** über die Kontaktseite **(6),**
C) Entfernen der Transfereinrichtung **(2)** mit dem die Kontaminationen enthaltenden Medium **(5)** von der kontaminierten Oberfläche,
D) Verbinden der Kontaktseite **(6)** des die Kontaminationen enthaltenden Mediums **(5)** in der Transfereinrichtung **(2)** über den zweiten Fixierrand **(7b)** mit der Analyseeinrichtung **(11),**
E) Ablösen des nach Schritt D) über die Kontaktseite **(6)** mit der Analyseeinrichtung **(11)** verbundenen Mediums **(5)** von dem Träger **(3)** der Transfereinrichtung **(2)** und
F) Analysieren des die Kontaminationen enthaltenden Mediums **(5),** welches mit der Analyseeinrichtung **(11)** verbunden ist,
wobei das Ablösen in Schritt E) durch eine durch den zweiten Fixierrand **(7b)** vermittelte Klebeverbindung ausgelöst wird, die eine größere Haftkraft auf das Medium **(5)** ausübt als eine durch den ersten Fixierrand **(7a)** vermittelte Klebeverbindung.

12. Verfahren nach Anspruch 11,
wobei zwischen dem Träger **(3)** und dem Medium **(5)** eine poröse Unterstützungsschicht **(4)** angeordnet ist, welche vor Schritt A) oder nach Schritt B) mit einer Analyseflüssigkeit oder mit einer Behandlungsflüssigkeit für eine Folgebehandlung des Mediums getränkt wird.

13. Verfahren nach einem der Ansprüche 11 oder 12,
wobei als poröses Medium **(5)** ein Membranfilter verwendet wird und die Kontaminationen Mikroben sind.

14. Verfahren nach Anspruch 13,
wobei zusätzlich in Schritt F) das Aufsetzen des über den zweiten Fixierrand **(7b)** mit der Analyseeinrichtung **(11)** verbundenen porösen Mediums **(5)** auf die Oberfläche eines Nährbodens in einem Unterteil eines Nährmedienbehälters erfolgt.

## Claims

1. Device (1) for analysing a contaminated surface, comprising
- a transfer device (2)
with a support (3) and
with a porous disc-shaped medium (5) with a contact side (6) able to be arranged on the contact surface,
wherein the support (3) is connectible by way of a first fixing edge (7a) with the medium (5) on the side (8) thereof remote from the contact side (6), and
- an analysing device (11),
which for removal of the medium (5) from the support (3) is connectible by way of a second fixing edge (7b) with the medium (5) on the contact side (6) thereof,
wherein the first fixing edge (7a) is connectible by way of a first adhesive connection and the second fixing edge (7b) by way of a second adhesive connection with the medium (5) and wherein the first adhesive connection is releasable by a lower action of force than the second adhesive connection.

2. Device (1) according to claim 1, wherein a support layer (4) is arranged between the support (3) and the medium (5) and wherein for removal of the medium (5) from the support layer (4) the analysing device (11) is connected by way of the second fixing edge (7b) with the medium (5) on the contact side (6) thereof.

3. Device (1) according to one of the preceding claims, wherein the first and second adhesive connections are formed to be temporary or reversible.

4. Device (1) according to any one of the preceding claims, wherein the first and second fixing edges (7a, 7b) respectively comprise an adhesive layer of an adhesive which is a pressure-sensitive dispersion adhesive or formed from acrylate-copolymer microspheres.

5. Device (1) according to claim 4, wherein the adhesive is applied to the first and/or second fixing edge (7a, 7b) by way of a water-soluble intermediate layer.

6. Device (1) according to any one of the preceding claims 2 to 5, wherein the support layer (4) is porous and consists of a resilient plastics material foam.

7. Device (1) according to claim 6, wherein the support layer (4) is saturated with an analysis liquid or with a treatment liquid for downstream treatment of the porous medium (5).

8. Device (1) according to any one of the preceding claims, wherein the second fixing edge (7b) is formed by a free end surface of an annular wall (12), which is arranged at a cover inner surface of the analysing device (11).

9. Device (1) according to any one of the preceding claims, wherein the analysing device (11) has an opening (13) which is closable by a removable cover (14) and by way of which downstream treatment of the medium (5) can be carried out.

10. Device (1) according to any one of the preceding claims 2 to 9, wherein the transfer device (2) is constructed as a component of a culture medium container, wherein the support layer (4) of the transfer device (2) is mounted on the underside of the culture medium container by way of an irreversible or a reversible adhesive connection.

11. Method of analysing a contaminated surface with use of a device (1) according to any one of claims 1 to 10, comprising the steps:
A) arranging the contact side (6) of the porous disc-shaped medium (5) of the transfer device (2) on the contaminated surface, wherein the medium (5) on its side (8) remote from the contact side (6) is connected by way of the first fixing edge (7a) with the support (3) of the transfer device (2),
B) taking up contaminants of the contaminated surface into the medium (5) via the contact side (6),
C) removing the transfer device (2) with the medium (5), which contains the contaminants, from the contaminated surface,
D) connecting the contact side (6) of the medium (5), which contains the contaminants, in the transfer device (2) by way of the second fixing edge (7b) with the analysing device (11),
E) detaching the medium (5), which after step D) is connected by way of the contact side (6) with the analysing device (11), from the support (3) of the transfer device (2) and
F) analysing the medium (5), which contains the contaminants and which is connected with the analysing device (11),
wherein the detaching in step E) is triggered by an adhesive connection which is produced by the second fixing edge (7b) and which exerts a greater adhesive force on the medium (5) than an adhesive connection produced by the first fixing edge (7a).

12. Method according to claim 11, wherein arranged between the support (3) and the medium (5) is a porous support layer (4) which before step A) or after step B) is saturated with an analysis liquid or with a treatment liquid for downstream treatment of the medium.

13. Method according to one of claims 11 and 12, wherein a membrane filter is used as porous medium (5) and the contaminants are microbes.

14. Method according to claim 13, wherein in addition in step F) the placing of the porous medium (5), which is connected by way of the second fixing edge (7b) with the analysing device (11), is carried out on the surface of a culture medium in a lower part of a culture medium container.

## Revendications

1. Dispositif (1) pour l'analyse d'une surface contaminée,
comprenant
- un système de transfert (2)
avec un support (3) et
avec un milieu (5) poreux sous la forme d'un disque comportant une face de contact (6) pouvant être disposée sur la surface contaminée,
le support (3) pouvant être relié avec le milieu (5) par sa face (8) opposée à la face de contact (6) par un premier bord de fixation (7a), et
- un système d'analyse (11),
qui peut être relié avec le milieu (5) au niveau de sa face de contact (6) par un deuxième bord de fixation (7b), pour l'enlèvement du milieu (5) du support (3),
le premier bord de fixation (7a) pouvant être relié avec le milieu (5) par un premier assemblage collé et le deuxième bord de fixation (7b) par un deuxième assemblage collé, et le premier assemblage collé pouvant être détaché par une force de sollicitation moindre que le deuxième assemblage collé.

2. Dispositif (1) selon la revendication 1,
où une couche de renfort (4) est disposée entre le support (3) et le milieu (5) et où le système d'analyse (11) peut être relié avec le milieu (5) sur sa face de contact (6) par le deuxième bord de fixation (7b) pour l'enlèvement du milieu (5) de la couche de renfort (4).

3. Dispositif (1) selon l'une des revendications précédentes,
dans lequel les premier et le deuxième assemblages collés sont conçus temporaires ou réversibles.

4. Dispositif (1) selon l'une des revendications précédentes,
dans lequel les premier et deuxième bords de fixation (7a, 7b) présentent chacun une couche d'adhésion à base de colle qui est un agent de collage en dispersion sensible à la pression ou un agent constitué de microbilles de copolymère d'acrylate.

5. Dispositif (1) selon la revendication 4,
dans lequel la colle est rapportée sur le premier et/ou le deuxième bord de fixation (7a, 7b) par une couche intermédiaire soluble dans l'eau.

6. Dispositif (1) selon l'une des revendications précédentes 2 - 5,
dans lequel la couche de renfort (4) est poreuse et est constituée d'une mousse synthétique élastique.

7. Dispositif (1) selon la revendication 6,
dans lequel la couche de renfort (4) est imprégnée avec un liquide d'analyse ou avec un liquide de traitement pour un traitement subséquent du milieu (5) poreux.

8. Dispositif (1) selon l'une des revendications précédentes,
dans lequel le deuxième bord de fixation (7b) est formé par une surface frontale libre d'une paroi (12) en forme d'anneau, laquelle est disposée sur une surface interne du couvercle du système d'analyse (11).

9. Dispositif (1) selon l'une des revendications précédentes,
dans lequel le système d'analyse (11) présente un orifice (13), pouvant être fermé avec un couvercle (14) amovible, par lequel un traitement subséquent du milieu (5) peut être réalisé.

10. Dispositif (1) selon l'une des revendications précédentes 2 à 9, dans lequel le système de transfert (2) est conçu comme un composant d'un récipient de milieu de nutrition, la couche de renfort (4) du système de transfert (2) étant rapportée sur la face inférieure du récipient de milieu nutritif par une liaison adhésive irréversible ou réversible.

11. Procédé pour l'analyse d'une surface contaminée moyennant l'utilisation d'un dispositif (1) selon l'une des revendications 1 à 10,
comprenant les étapes :
A) de disposition de la face de contact (6) du milieu (5) poreux sous forme de disque du système de transfert (2) sur la surface contaminée, le milieu (5) étant relié avec le support (3) du système de transfert (2) par sa face opposée (8) à la face de contact (6) par le premier bord de fixation (7a),
B) d'absorption des contaminations à partir de la surface contaminée dans le milieu (5) par la face de contact (6),
C) d'enlèvement de la surface contaminée du système de transfert (2) comprenant le milieu (5) contenant les contaminations,
D) de mise en relation de la face de contact (6) du milieu (5) contenant les contaminations dans le système de transfert (2) avec le système d'analyse (11) par le deuxième bord de fixation (7b),
E) de détachement du milieu (5), relié selon l'étape D) avec le système d'analyse (11) par sa face de contact (6), du support (3) du système de transfert (2), et
F) d'analyse du milieu (5) contenant les contaminations, lequel est relié avec le système d'analyse (11),
le détachement dans l'étape E) étant résolu par un assemblage collé réalisé à l'aide du deuxième bord de fixation (7b), qui exerce une force d'adhésion supérieure à une force conférée par le premier bord de fixation (7a) sur le milieu (5).

12. Procédé selon la revendication 11,
dans lequel une couche de renfort (4) poreuse est disposée entre le support (3) et le milieu (5), laquelle est imprégnée avec un liquide d'analyse ou un liquide de traitement avant l'étape (A) ou après l'étape B) pour un traitement subséquent du milieu.

13. Procédé selon l'une des revendications 11 ou 12,
dans lequel un filtre membranaire est utilisé en tant que milieu (5) poreux et où les contaminations sont des microbes.

14. Procédé selon la revendication 13,
dans lequel, en complément dans l'étape F), la mise en place du milieu (5) poreux relié avec le système d'analyse (11) par le deuxième bord de fixation (7b) sur la surface d'un support nutritif est effectuée dans une partie inférieure du récipient du milieu nutritif.
